(19) Europäisches Patentamt
European Patent Office
Office européen des brevets

(11) **EP 3 496 734 B1**

(12) **EUROPEAN PATENT SPECIFICATION**

(45) Date of publication and mention
of the grant of the patent:
**15.12.2021 Bulletin 2021/50**

(51) Int Cl.:
*A61K 38/00* (2006.01)     *A61K 38/28* (2006.01)
*A61K 47/18* (2017.01)     *A61K 9/00* (2006.01)
*A61P 3/10* (2006.01)

(21) Application number: **17754468.1**

(22) Date of filing: **11.08.2017**

(86) International application number:
**PCT/GB2017/052377**

(87) International publication number:
**WO 2018/029489 (15.02.2018 Gazette 2018/07)**

(54) **INSULIN GLARGINE**

INSULIN GLARGINE

INSULINE GLARGINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2016 GB 201613895**

(43) Date of publication of application:
**19.06.2019 Bulletin 2019/25**

(73) Proprietor: **Arecor Limited
Little Chesterford
Saffron Walden
CB10 1XL (GB)**

(72) Inventors:
• **GERRING, David
Saffron Walden
CB10 1XL (GB)**
• **HOWELL, Sarah
Saffron Walden
CB10 1XL (GB)**

• **JEZEK, Jan
Saffron Walden
CB10 1XL (GB)**
• **ZAKRZEWSKI, Leon
Saffron Walden
CB10 1XL (GB)**
• **WATTS, Gary
Saffron Walden
CB10 1XL (GB)**

(74) Representative: **O'Kane, Jessica Ann
Sagittarius IP
Marlow International
Parkway
Marlow SL7 1YL (GB)**

(56) References cited:
**WO-A1-2017/145104      US-A1- 2008 039 368
US-A1- 2011 230 402      US-A1- 2015 182 595
US-A1- 2015 273 022**

SEQUENCE LISTING

<110> Arecor Ltd

<120> Novel Composition

<130> ACR-P2037PCT

<160> 2

<170> PatentIn version 3.5

<210> 1
<211> 21
<212> PRT
<213> Homo sapiens

<400> 1

```
        Gly Ile Val Glu Gln Cys Cys Thr Ser Ile Cys Ser Leu Tyr Gln Leu
        1               5                   10                  15


        Glu Asn Tyr Cys Gly
                    20
```

<210> 2
<211> 32
<212> PRT
<213> Homo sapiens

<400> 2

```
Phe Val Asn Gln His Leu Cys Gly Ser His Leu Val Glu Ala Leu Tyr
 1               5               10                  15
```

```
Leu Val Cys Gly Glu Arg Gly Phe Phe Tyr Thr Pro Lys Thr Arg Arg
         20                   25                  30
```

**Description**

This invention relates to aqueous solution compositions of insulin glargine and their use in therapy, particularly in applications where chronic administration of insulin glargine is desirable.

Background

The current insulin market comprises a number of different products, including regular human insulin (RHI) and a number of insulin analogues. The analogues have been developed by amino acid substitutions to provide certain advantages over RHI. In particular, the advantages relate to the speed of action of insulin. Some analogues (e.g. insulin lispro, insulin aspart and insulin glulisine) have been developed to provide a faster onset of action and are therefore the preferred option for prandial use (i.e. before/with meal). These are referred to as 'rapid acting insulin analogues'. In contrast, other analogues such as insulin glargine and insulin degludec have been developed to act very slowly and are thus used as basal insulins providing basal glycaemic control for -24 hours period. These are referred to as 'long acting insulin analogues' or 'slow acting insulin analogues' or 'basal insulins'.

Insulin glargine is a basal insulin analogue resulting from the substitution of a glycine residue at position 21 of the A-chain of an insulin molecule for asparagine and the addition of two arginine residues to the B-chain at position 30 (and is sometimes represented as Gly(A21),Arg(B31),Arg(B32)-human insulin). These structural modifications cause a shift in the isoelectric point of the molecule, rendering it more soluble at an acidic pH and significantly decreasing its solubility at physiological pH. Insulin glargine is typically formulated at pH 4 in the absence of a buffer. Following injection into the (pH-neutral) subcutaneous tissue, insulin glargine forms microprecipitates from which it is subsequently slowly released (http://www.lantus.com/hcp/about-lantus/how-lantus-works). The precipitation is a direct consequence of pH change from 4 to -7.4 (physiological pH). This slow release ensures that small amounts of insulin glargine are released into the body continuously, giving an almost peakless profile.

Insulin glargine has been marketed as a 100 U/ml product by Sanofi Aventis under the brand name Lantus®. More recently, Sanofi Aventis started marketing a 300 U/ml insulin glargine product under the brand name Toujeo®, which was shown to have a more prolonged PK/PD profile compared with Lantus®, resulting in longer glucose control. In addition, a biosimilar version of insulin glargine (100 U/ml) has been launched by Eli Lilly under the brand name Abasaglar®.

Lantus® is marketed both in a pen presentation and in a multi-dose vial presentation. The composition of Lantus® for a pen cartridge is as follows: 100 U/ml insulin glargine (equivalent to approximately 3.6 mg/ml); 25 mM m-cresol (a preservative); 185 mM glycerol and 30 μg/ml ionic zinc (from zinc oxide or zinc chloride); at pH 4.0.

The composition of Lantus® for vials is identical to that for the pen cartridge, except that it also contains polysorbate 20 (20 μg/ml), which is added to the vial formulation in order to prevent agitation-related aggregation. Agitation-related aggregation is a significant concern for the vial presentation due to the presence of a headspace, but not for the pen cartridge where there is no headspace.

Abasaglar® is currently only available in a pen presentation and the composition is identical to that of Lantus®.

Toujeo® (containing 300 U/ml insulin glargine) is formulated together with the same components at the same concentrations as for Lantus® with the exception of the ionic zinc concentration - the insulin/zinc (w/w) ratio maintained is identical to that of the Lantus® product, so the zinc level is 90 μg/ml in Toujeo®.

WO2010/149772 (NOVO NORDISK) discloses insulin preparations comprising an insulin compound or a mixture of two or more insulin compounds, a nicotinic compound and an amino acid.

WO2014/096985 (WOCKHARDT LIMITED) discloses insulin preparations comprising human insulin, analogues or derivatives thereof, and one or more solubility enhancing agents selected from urea, amino acids and/or surfactants.

US2015/273022 (BIODEL INC) discloses compositions and methods for enhancing the stability of rapid acting injectable insulin formulations.

US2008/039368 (BIODEL INC) discloses rapid acting and long acting insulin combination formulations wherein the pH of the rapid acting insulin is adjusted so that the long acting glargine remains soluble when they are mixed together.

US2011/230402 (NOVO NORDISK) discloses an insulin derivative which exhibits a prolonged profile of action.

WO2017/145104 (WOCKHARDT LIMITED) discloses insulin preparations comprising insulin glargine, at least two amino acids and optionally one or more pharmaceutically acceptable excipients.

The currently marketed insulin glargine products must be stored at 2-8 °C prior to use. During the 28 days in-use period (i.e. period starting from the first use of the pen cartridge or vial) the product can be kept at temperatures up to 30 °C (refrigerated or unrefrigerated at 15-30 °C is recommended).

In order to improve patients' convenience and shipment logistics there is a need for insulin glargine compositions with improved stability, in particular compositions wherein:

- the temperature at which the product can be kept during the in-use period is increased, e.g. to 40 °C;

- the duration of the in-use period is increased, e.g. to 2 months, preferably 3 months;

- the product can be stored at increased temperature, such as controlled room temperature (20-25 °C) for part of the shelf-life, e.g. 3 months, 6 months or the entire shelf-life, whilst maintaining the in-use stability at 30 °C for 28 days.

Thus, an object of the present invention is the provision of an aqueous solution composition of insulin glargine as active ingredient with improved stability.

Summary of the invention

Thus, according to the invention, there is provided an aqueous solution composition comprising insulin glargine as an active ingredient and an amino acid selected from aspartic acid and glutamic acid as a stabilising agent, wherein the amino acid is present at a concentration of 1-50 mM.

Figures

Fig. 1: Stability of insulin glargine (100 U/ml) in the presence of sodium chloride assessed by SEC following storage at 30 °C for 4 and 12 weeks (see Table 2 of Example 1).

Fig. 2: Stability of insulin glargine (100 U/ml) in the presence of sodium chloride assessed by RP-HPLC following storage at 30 °C for 4 and 12 weeks (see Table 3 of Example 1).

Fig. 3: Stability of insulin glargine (100 U/ml) in the presence of aspartic acid assessed by SEC following storage at 30 °C for 4 and 12 weeks (see Table 5 of Example 2).

Fig. 4: Stability of insulin glargine (100 U/ml) in the presence of aspartic acid assessed by RP-HPLC following storage at 30 °C for 4 and 12 weeks (see Table 6 of Example 2).

Fig. 5: Stability of insulin glargine (100 U/ml) in the presence of aspartic acid assessed by SEC following storage at 30 °C for 4 and 8 weeks (see Table 8 of Example 3).

Fig. 6: Stability of insulin glargine (100 U/ml) in the presence of glutamic acid assessed by SEC following storage at 30 °C for 4 and 8 weeks (see Table 8 of Example 3).

Fig. 7: Stability of insulin glargine (100 U/ml) in the presence of glycine assessed by SEC following storage at 30 °C for 4 and 8 weeks (see Table 8 of Example 3).

Fig. 8: Stability of insulin glargine (100 U/ml) in the presence of aspartic acid assessed by RP-HPLC following storage at 30 °C for 4 and 8 weeks (see Table 9 of Example 3).

Fig. 9: Stability of insulin glargine (100 U/ml) in the presence of glutamic acid assessed by RP-HPLC following storage at 30 °C for 4 and 8 weeks (see Table 9 of Example 3).

Fig. 10: Stability of insulin glargine (100 U/ml) in the presence of glycine assessed by RP-HPLC following storage at 30 °C for 4 and 8 weeks (see Table 9 of Example 3).

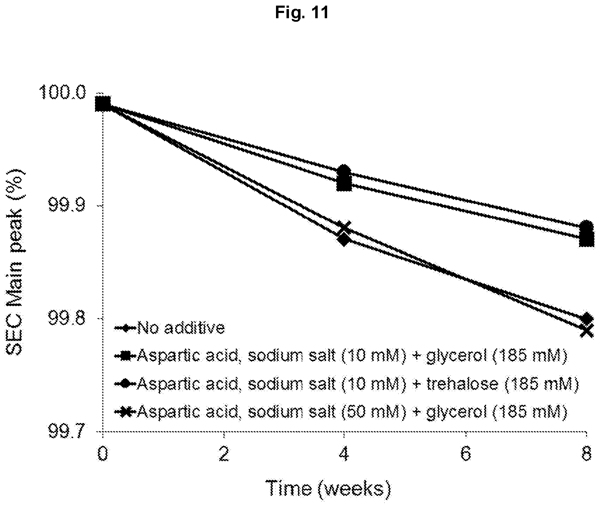

Fig. 11: Stability of insulin glargine (500 U/ml) in the presence of aspartic acid assessed by SEC following storage at 30 °C for 4 and 8 weeks (see Table 11 of Example 4).

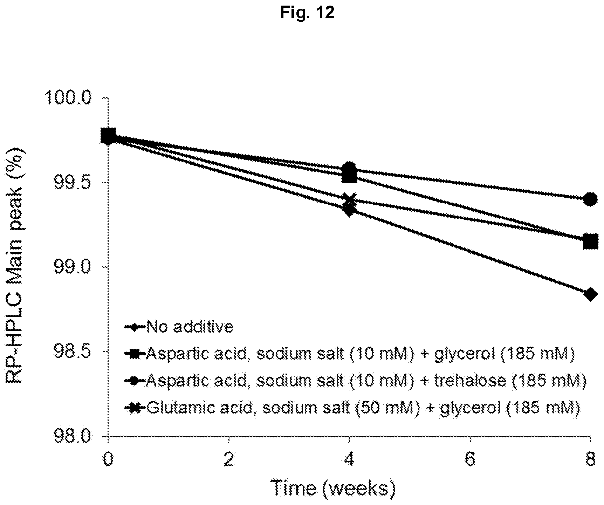

Fig. 12: Stability of insulin glargine (500 U/ml) in the presence of aspartic acid assessed by RP-HPLC following storage at 30 °C for 4 and 8 weeks (see Table 12 of Example 4).

Description of the sequence listing

SEQ ID NO. 1: A-chain of insulin glargine

SEQ ID NO. 2: B-chain of insulin glargine

Detailed description of the invention

As used herein "insulin glargine" refers to the analogue of native human insulin having an A-chain as set out in SEQ ID NO. 1 and a B-chain as set out in SEQ ID NO. 2, and containing and connected by disulfide bridges as in the native molecule (Cys A6-Cys A11, Cys B7 to Cys A7 and Cys-B19-Cys A20). Reference to a "composition" herein is intended to refer to an aqueous solution composition.

The concentration of insulin glargine in the composition is typically between 10 U/ml and 1000 U/ml, for example between 50 U/ml and 500 U/ml, or between 100 U/ml and 200 U/ml. Alternatively the concentration of insulin glargine in the composition may be between 200 U/ml and 500 U/ml or between 500 U/ml and 700 U/ml. In one embodiment, the concentration of insulin glargine is about 100 U/ml. In one embodiment, the concentration of insulin glargine is about 200 U/ml. In another embodiment, the concentration of insulin glargine is about 300 U/ml. In another embodiment, the concentration of insulin glargine is about 400 U/ml. In another embodiment, the concentration of insulin glargine is about 500 U/ml. "U/ml" as used herein describes the concentration of insulin glargine in terms of a unit per volume, wherein "U" describes the activity of insulin glargine that is biologically equivalent to 34.7 $\mu$g of pure crystalline insulin. 100 U corresponds to 3.64 mg of insulin glargine.

The composition of the invention includes an amino acid selected from aspartic acid and glutamic acid as a stabilising agent. The amino acid can be added to the composition in salt form, for example as the mono-sodium salt, e.g. sodium aspartate. The amino acid can also be added to the composition in pure form, i.e. without any counterions. In one embodiment, the amino acid is aspartic acid. In another embodiment, the amino acid is glutamic acid. In a further embodiment, the amino acid is a mixture of aspartic acid and glutamic acid. Thus, reference to "an amino acid" in this context also includes mixtures of aspartic acid and glutamic acid.

The amino acid is present at a concentration of 1-50 mM, for example 2-45 mM, 5-45 mM, 2-40 mM, 5-40 mM or 2-25 mM such as 5-15 mM, 6-14 mM, 7-12 mM, 7-13 mM, 8-12 mM, such as about 10 mM. When a mixture of aspartic acid and glutamic acid is used, the aforementioned concentration ranges refer to the total (i.e. combined) concentration of amino acid (aspartic acid and glutamic acid) present.

The pH of the composition is suitably between 3 and 5, for example between 3.5 and 4.5. Preferably it is around pH 4. Insulin glargine in a composition at such a pH is typically completely soluble.

The composition may additionally comprise a tonicity modifier, which may be charged or uncharged. Examples of uncharged tonicity modifiers include glycerol, 1,2-propanediol, mannitol, sorbitol, trehalose, PEG300 and PEG400. When included, an uncharged tonicity modifier is typically added at a concentration of 50-1000 mM, for example 100-500 mM, such as about 300 mM. Examples of charged tonicity modifiers include sodium chloride, sodium sulfate, and amino acids such as glycine or arginine. When included, a charged tonicity modifier is typically added at a concentration of 25-500 mM, for example 50-250 mM such as about 150 mM. An uncharged tonicity modifier rather than a charged tonicity modifier is generally preferred.

The composition may additionally comprise a surfactant. In one embodiment, the surfactant is a non-ionic surfactant. In another embodiment, the surfactant is a cationic surfactant. Suitable cationic surfactants include benzalkonium and benzethonium salts. In one embodiment, the cationic surfactant is selected from benzethonium salts e.g. benzethonium halide such as benzethonium chloride. In another embodiment, the cationic surfactant is selected from benzalkonium salts e.g. benzalkonium halide such as benzalkonium chloride. In a further embodiment, the cationic surfactant is a mixture of benzethonium salts and benzalkonium salts such as a mixture of benzethonium chloride and benzalkonium chloride.

When included, the surfactant is typically at a concentration of 5-200 $\mu$M, such as 10-100 $\mu$M or 20-100 $\mu$M.

The compositions of the invention may additionally comprise a preservative such as a phenolic or a benzylic preservative. The preservative is suitably selected from the group consisting of phenol, m-cresol, chlorocresol, benzyl alcohol, propyl paraben and methyl paraben, in particular phenol, m-cresol and benzyl alcohol.

The concentration of preservative is typically 1-100 mM, for example 20-80 mM, such as 25-50 mM. The optimal concentration of the preservative in the composition is selected to ensure the composition passes the Pharmacopoeia Antimicrobial Effectiveness Test (USP <51>, Vol. 32).

Both the aspartic acid and/or glutamic acid will act as buffers at the preferred pH range of between 3 and 5. However, the composition may comprise an additional buffer. Suitable buffers include acetate, succinate, citrate, histidine, malate and maleate.

The composition suitably comprises ionic zinc i.e. $Zn^{2+}$ cations. The source of ionic zinc will typically be a water soluble zinc salt such as $ZnCl_2$, $ZnO$, $ZnSO_4$, $Zn(NO_3)_2$ or $Zn(acetate)_2$ and is most suitably $ZnCl_2$ or $ZnO$. Suitably, the amount of ionic zinc present is between 10-100 $\mu$g per 100 U of insulin glargine, such as 20-50 $\mu$g, e.g. around 30 $\mu$g per 100 U of insulin glargine. The amount of ionic zinc in the concentration calculation does not include the mass of the counter ion.

Suitably the overall concentration of charged species in the composition is low. In the context of this invention, a charged species is defined as a chemical entity which carries at least one charge under the conditions of the composition, e.g. sodium cation ($Na^+$), chloride anion ($Cl^-$) or an amino acid such as histidine. Suitably, the overall concentration of charged species, other than those originating from ionic zinc, aspartic acid and/or glutamic acid and insulin glargine in the composition is less than 150 mM, for example less than 100 mM, such as less than 50 mM or less than 25 mM. In one embodiment the composition is substantially free of ionic species (apart from insulin glargine, ionic zinc and aspartic acid and/or glutamic acid) which possess more than one charged group or which have a total charge of more than 1. For example, the composition is substantially free of ionic species (apart from insulin glargine, ionic zinc and aspartic acid and/or glutamic acid) which possess 2, 3, 4 or more charged groups. For example, the composition is substantially free of ionic species (apart from insulin glargine, ionic zinc and aspartic acid and/or glutamic acid) which have a total charge of 2, 3, 4 or more.

The ionic strength of a composition may be calculated according to the formula:

$$I = 0.5 \times \sum_{X=1}^{n} c_x z_x^2$$

in which $c_x$ is molar concentration of ion x (mol $L^{-1}$), $z_x$ is the absolute value of the charge of ion x and the sum covers all ions (n) present in the composition. The contribution of insulin glargine itself and of the ionic zinc, and of the aspartic acid and/or glutamic acid should be ignored for the purposes of the calculation. The ionic strength of the composition is suitably kept to a minimum level since higher ionic strength compositions are typically less stable than comparable lower ionic strength compositions. Suitably the total ionic strength of the composition is less than 40 mM, e.g. less than 20 mM, e.g. less than 10 mM.

In one embodiment, the composition of the invention does not contain a nicotinic compound. In one embodiment, the composition of the invention does not contain glycine. In one embodiment, the composition of the invention does not contain arginine. In one embodiment, the composition of the invention does not contain histidine.

A specific embodiment that may be envisaged is a composition according to the invention with a pH of about 4, comprising insulin glargine as an active ingredient at a concentration of between 50 U/mL and 500 U/mL, aspartic acid and/or glutamic acid as a stabilising agent at a total concentration of 2-45 mM e.g. 5-40 mM, and an uncharged tonicity modifier selected from glycerol, 1,2-propanediol, mannitol, sorbitol, trehalose, PEG300 and PEG400.

The presently claimed invention derives from the surprising observation that compositions of insulin glargine as an active ingredient are stabilized by the addition of amino acids aspartic acid and glutamic acid at a concentration of 1-50 mM. An improvement in both chemical and physical stability is observed (see Examples 2-4).

This result was surprising in view of the inventors' observation that increasing the ionic strength of insulin glargine composition results in a decrease in physical and chemical stability (see Example 1). At the acidic pH typically used to formulate insulin glargine, both of the amino acids aspartic acid and glutamic acid are charged, and would therefore increase the ionic strength of the composition. Based on the observations of Example 1, it would be expected that an increase in ionic strength would result in no change or a decrease in stability.

As can be seen from Example 3, the addition of the amino acid glycine resulted in comparable or only very slightly improved stability. However, the addition of amino acids aspartic acid and glutamic acid each produced a notable enhancement in stability. Without wishing to be bound by theory, it appears that the claimed composition provides optimum components and concentrations for the stability of insulin glargine, resulting from a balance between a stabilizing effect of aspartic acid and glutamic acid, and the destabilizing effect of increasing the ionic strength of the composition.

As shown in Examples 2 and 3, compositions of the invention demonstrated good stability following storage at 30 °C for 4 weeks, 8 weeks and 12 weeks.

Enhanced stability was also observed for higher concentrations of insulin glargine (500 U/ml), as shown in Example 4.

The physical stability of an insulin glargine composition refers to the tendency of the insulin glargine molecule to form insoluble aggregates due to, *inter alia,* destabilizing interactions with surfaces and interfaces, and temperature fluctuations. Aggregates are described herein as "high molecular weight species", which refers to any irreversibly formed component of protein content which has an apparent molecular weight at least double the molecular weight of the parent insulin glargine molecule. Thus, high molecular weight species are multimeric aggregates of the parent insulin glargine molecule, which may comprise the parent insulin glargine molecules with considerably altered conformation or they may be an assembly of the parent insulin glargine units in the native or near-native conformation. Physical stability of the insulin glargine composition can be evaluated by methods known in the art, including by visual inspection, size exclusion chromatography (SEC), electrophoresis, analytical ultracentrifugation, light scattering, dynamic light scattering, static light scattering and field flow fractionation. Exemplary methods using visual inspection and size exclusion chromatography (SEC) are described in the General Procedures.

The chemical stability of an insulin glargine composition refers to changes in the covalent protein structure of the insulin glargine molecule leading to the formation of chemically related insulin glargine species (degradation products). Chemical stability of the insulin glargine composition can be evaluated by reversed phase HPLC (RP-HPLC) to determine the proportion of total chemically related insulin glargine species (i.e. species generated during storage or other stress conditions by chemical modification of insulin glargine, including, deamidation of glutamine or asparagine residues, cyclic imide formation or various hydrolysis processes), as described in the General Procedures.

Suitably the composition of the invention remains as a clear solution for a longer period of time compared to the current commercially available insulin glargine products, allowing for a longer in-use period. Thus, in one embodiment, the composition of the invention remains as a clear solution during storage at 15-30 °C e.g. at 30 °C for longer than 4 weeks, for example for 5 weeks, 6 weeks, 7 weeks, 8 weeks, 10 weeks or 12 weeks. By "clear solution" it is meant that no visible precipitation is observed during storage.

Suitably the composition of the invention remains as a clear solution when exposed to temperatures which are higher than those recommended for the current commercially available insulin glargine products. Thus, in one embodiment, the composition of the invention remains as a clear solution during storage for 4 weeks, at temperatures greater than 30 °C, for example during storage for 4 weeks at 32 °C, at 34 °C, at 35 °C, at 37 °C, at 38 °C or at 40 °C.

Preferably, the composition of the invention provides a more user-friendly in-use period in that the composition can be stored both at higher temperatures, and for longer periods of time, than recommended for the current commercially available insulin glargine products. Thus, in one embodiment, the composition of the invention remains as a clear solution during storage at 32 °C or higher, for example at 32 °C, 34 °C, at 35 °C, at 37 °C, at 38 °C or at 40 °C; for at least 4 weeks, for example for 4 weeks, 5 weeks, 6 weeks, 7 weeks, 8 weeks, 10 weeks or 12 weeks.

In one embodiment, the composition according to the invention is a clear solution with low viscosity (e.g. dynamic viscosity of less than 20 cP, such as less than 10 cP, e.g. less than 5 cP at 25 °C measured using a microfluidics capillary extrusion viscometer, such as m-VROC™, RheoSense Inc.).

Suitably the composition of the invention has improved storage stability at increased temperature, while maintaining the in-use stability. Thus, in one embodiment, the composition of the invention remains as a clear solution during storage at 25 °C for at least 3 months, for example 3 months, 6 months, 12 months or 24 months; and also remains as a clear solution during an in-use period of 28 days at 30 °C, starting immediately after the end of the storage period.

In one embodiment, the composition of the invention comprises no more than 4% (by total weight of insulin glargine in the composition) of high molecular weight species, preferably no more than 3%, 2%, or 1%, following storage at 15-30 °C e.g. 30 °C, for longer than 4 weeks, for example 5 weeks, 6 weeks, 7 weeks, 8 weeks, 10 weeks or 12 weeks. By "clear solution" it is meant that no visible precipitation is observed during storage.

In one embodiment, the composition of the invention comprises no more than 4% (by total weight of insulin glargine in the composition) of high molecular weight species, preferably no more than 3%, 2% or 1%, following storage for at least 4 weeks, at temperatures greater than 30 °C, for example during storage for 4 weeks at 32 °C, 34 °C, 35 °C, 37 °C, 38 °C or 40 °C.

In one embodiment, the composition of the invention comprises no more than 4% (by total weight of insulin glargine in the composition) of high molecular weight species, preferably no more than 3%, 2% or 1%, following storage at 32 °C or higher, for example 32 °C, 34 °C, 35 °C, 37 °C, 38 °C or 40 °C; for at least 4 weeks, for example for 4 weeks, 5 weeks, 6 weeks, 7 weeks, 8 weeks, 10 weeks or 12 weeks.

In one embodiment, the composition of the invention comprises no more than 4% (by total weight of insulin glargine in the composition) of high molecular weight species, preferably no more than 3%, 2%, or 1% following storage at 25 °C for at least 3 months, such as 3 months, 6 months, 12 months or 24 months; and also comprises no more than 4% (by total weight of insulin glargine in the composition) of high molecular weight species, preferably no more than 3%, 2% or 1% following an in-use period of 28 days at 30 °C, starting immediately after the end of the storage period.

In one embodiment, a composition of the present invention exhibits an increase in high molecular weight species during storage which is at least 10% lower, preferably at least 25% lower, more preferably at least 50% lower, than a composition lacking the aspartic acid and/or glutamic acid as stabilising agent at a concentration of 1-50 mM, but otherwise identical, following storage under the same conditions and length of time.

Suitably, a composition of the invention retains at least 95%, e.g. at least 96%, at least 97%, at least 98% or at least 99% native insulin glargine (by total weight of insulin glargine in the composition at time T = 0) following storage at 15-30 °C e.g. 30 °C, for at least 4 weeks, for example for 4 weeks, 5 weeks, 6 weeks, 7 weeks, 8 weeks, 10 weeks or 12 weeks.

Suitably, a composition of the invention retains at least 95%, e.g. at least 96%, at least 97%, at least 98% or at least 99% native insulin glargine (by total weight of insulin glargine in the composition at time T = 0) following storage for at least 4 weeks, at temperatures greater than 30 °C, for example during storage for 4 weeks at 32 °C, 34 °C, 35 °C, 37 °C, 38 °C or 40 °C.

Suitably, a composition of the invention retains at least 95%, e.g. at least 96%, at least 97%, at least 98% or at least 99% native insulin glargine (by total weight of inulin glargine in the composition at time T = 0) following storage at 32 °C or higher, for example at 32 °C, 34 °C, 35 °C, 37 °C, 38 °C or 40 °C, for at least 4 weeks, for example for 4 weeks, 5 weeks, 6 weeks, 7 weeks, 8 weeks, 10 weeks or 12 weeks.

Suitably, a composition of the invention retains at least 95%, e.g. at least 96%, at least 97%, at least 98% or at least 99% native insulin glargine (by total weight of insulin glargine in the composition at time T = 0) following storage at 25 °C for at least 3 months, such as 3 months, 6 months, 12 months or 24 months; and also retains at least 95%, e.g. at least 96%, at least 97%, at least 98% or at least 99% native insulin glargine (by total weight of insulin glargine in the composition at time T = 0) following an in-use period of 28 days at 30 °C, starting immediately after the end of the storage period.

Suitably, a composition of the present invention should exhibit an increase in chemically related insulin glargine species during storage which is at least 10% lower, preferably at least 25% lower, more preferably at least 50% lower, than a composition lacking the aspartic acid and glutamic acid as stabilising agent at a concentration of 1-50 mM, but otherwise identical, following storage under the same conditions and length of time.

Suitably, the proportion of total chemically related insulin glargine species remains below 4% (by weight), preferably below 3%, more preferably below 2%, and even more preferably below 1% during storage at 15-30 °C e.g. 30 °C, for at least 4 weeks, for example 4 weeks, 5 weeks, 6 weeks, 7 weeks, 8 weeks, 10 weeks or 12 weeks.

Suitably, the proportion of total chemically related glargine species remains below 4% (by weight), preferably below 3%, more preferably below 2%, and even more preferably below 1%, during storage for at least 4 weeks, at temperatures greater than 30 °C, for example during storage for 4 weeks at 32 °C, 34 °C, 35 °C, 37 °C, 38 °C or 40 °C.

Suitably, the proportion of total chemically related insulin glargine species remains below 4% (by weight), preferably below 3%, more preferably below 2%, and even more preferably below 1% during storage at least 32 °C or higher, for example at 32 °C, 34 °C, 35 °C, 37 °C, 38 °C or 40 °C, for at least 4 weeks, for example for 4 weeks, 5 weeks, 6 weeks, 7 weeks, 8 weeks, 10 weeks or 12 weeks.

Suitably, the proportion of total chemically related insulin glargine species remains below 4% (by weight), preferably below 3%, more preferably below 2%, and even more preferably below 1% following storage at 25 °C for 3 months, 6 months, 12 months or 24 months; and also remains below 4% (by weight), preferably below 3%, more preferably below 2%, and even more preferably below 1% following an in-use period of 28 days at 30 °C, starting immediately after the end of the storage period.

In another aspect of the invention, there is provided the use of an amino acid selected from aspartic acid and glutamic acid, at a concentration of 1-50 mM, for stabilizing an insulin glargine composition.

In a further aspect of the invention, there is provided a method of improving the stability of an aqueous solution composition comprising insulin glargine as an active ingredient which comprises adding an amino acid selected from aspartic acid and glutamic acid as a stabilising agent, at a concentration of 1-50 mM, to the composition.

The composition of the invention is a therapeutic composition.

Thus, in a further aspect of the invention is provided a composition as described herein for use as a pharmaceutical, especially for use in the treatment of diabetes mellitus. For example, said treatment of diabetes mellitus is chronic treatment.

A typical starting dose of a composition of the invention in patients with type 1 diabetes should be approximately one-third of the total daily insulin requirement. Depending on the size and condition this corresponds to 5-50 U, for example 5-25 U such as 15 U, typically administered once a day.

A typical starting dose of a composition of the invention in patients with type 2 diabetes is 0.2 U per kg of body weight, administered once daily, which should subsequently be adjusted to the patient's needs.

There is also provided a container, for example made of plastics or glass, containing one dose or a plurality of doses of the composition as described herein. The container can be for example, a vial, or a cartridge designed to be a replaceable item for use with an injection device.

The compositions of the invention may suitably be packaged for injection, especially sub-cutaneous or intramuscular injection. Sub-cutaneous injection is preferred. Injection may be by conventional syringe or more preferably via a pen device adapted for use by diabetic subjects. Exemplary pen devices include OptiClick®, SoloSTAR® and KwikPen®.

An aspect of the invention is an injection device, particularly a device adapted for subcutaneous or intramuscular injection, for single or multiple use comprising a container containing one dose or a plurality of doses of the composition of the invention together with an injection needle. In an embodiment, the container is a replaceable cartridge which contains a plurality of doses. In an embodiment, the needle is replaceable e.g. after each occasion of use. In one embodiment, the injection device is in the form of a pen.

Another aspect of the invention is a medical device comprising a reservoir comprising a plurality of doses of the composition of the invention and a pump adapted for automatic or remote operation such that upon automatic or remote operation one or more doses of the composition of the invention is administered to the body e.g. subcutaneously or intramuscularly. Such devices may be worn on the outside of the body or implanted in the body.

Insulin glargine-containing compositions according to the invention are expected to have one or more of the advantages of:

- good stability during the in-use period at temperatures up to 40 °C;
- good stability during an extended in-use period e.g. up to 12 weeks;
- good storage stability at an increased temperature e.g. 20-25 °C whilst retaining good in-use stability;

Compositions according to the invention are expected to have good physical and chemical stability as described herein.

## EXAMPLES

## General procedures

Materials

Insulin glargine used in Examples 1-3 was obtained from HEC Pharm. Insulin glargine used in Example 4 was obtained from a different supplier.

Analysis of insulin glargine physical stability

*Visual assessment*

Visible particles are suitably detected using the 2.9.20. European Pharmacepoeia Monograph (Particulate Contamination: Visible Particles). The apparatus required consists of a viewing station comprising:

- a matt black panel of appropriate size held in a vertical position
- a non-glare white panel of appropriate size held in a vertical position next to the black panel
- an adjustable lampholder fitted with a suitable, shaded, white-light source and with a suitable light diffuser (a viewing illuminator containing two 13 W fluorescent tubes, each 525 mm in length, is suitable). The intensity of illumination at the viewing point is maintained between 2000 lux and 3750 lux.

Any adherent labels are removed from the container and the outside washed and dried. The container is gently swirled or inverted, ensuring that air bubbles are not introduced, and observed for about 5 s in front of the white panel. The procedure is repeated in front of the black panel. The presence of any particles is recorded.

*Size Exclusion Chromatography (SEC)*

Ultra-high performance size exclusion chromatography of insulin glargine preparations is performed using the Waters ACQUITY H-class Bio UPLC® system with a 1.7 $\mu$m Ethylene Bridged Hybrid 125 Å pore packing material in a 300 mm by 4.6 mm column. The column is equilibrated in 0.65 mg/ml L-arginine, 20% v/v acetonitrile, 15% v/v glacial acetic acid mobile phase. Flow rate is 0.4 mL/min and UV detection (276 nm) is used. Injection volume is 10 $\mu$L. All analyses are performed at ambient temperature.

Analysis of insulin glargine chemical stability

*Reversed phase high-performance liquid chromatography (RP-HPLC)*

Ultra-high performance reverse phase chromatography is performed using the Waters ACQUITY H-class Bio UPLC® system with a 1.7 $\mu$m Ethylene Bridged Hybrid particle, 130 Å pore resin trifunctionally immobilised with a C18 ligand in a 50 mm by 2.1 mm column. Insulin samples are bound in an 82% w/v $Na_2SO_4$, 18% v/v acetonitrile, pH 2.3 mobile phase and eluted in 50% w/v $Na_2SO_4$, 50% v/v acetonitrile gradient flow. 2 $\mu$l of sample is acidified with 0.01M HCl and analysed at 0.61 mL/min, with 214 nm UV detection. All analyses are performed at 40 °C.

Example 1 - Effect of ionic strength (from inorganic salts) on stability of insulin glargine (100 U/ml)

The effect of ionic strength on the stability of insulin glargine (100 U/ml) was studied by comparing the stability of insulin glargine (100 U/ml) in the currently marketed formulation of Lantus® both in the absence and in the presence of specific concentrations of sodium chloride and sodium sulphate. The currently marketed formulation of Lantus® contains 25 mM m-cresol, 185 mM glycerol and 30 μg/ml ionic zinc and is adjusted to pH 4.0.

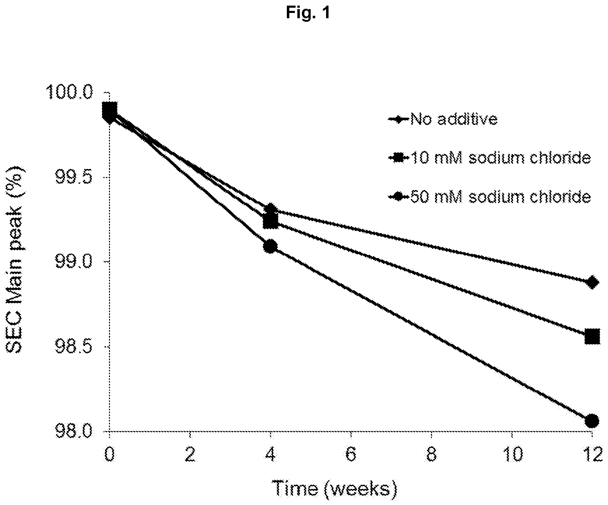

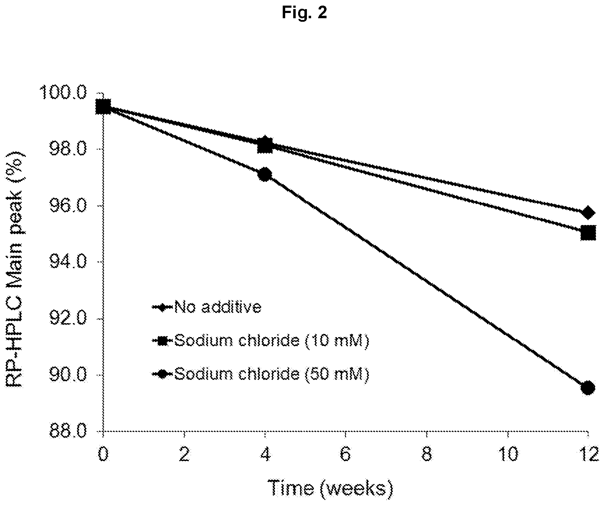

It was shown (Tables 1-3, Figures 1-2) that the addition of sodium chloride resulted in impairment of both physical stability (assessed by visual assessment and SEC (see General Procedures)) and chemical stability (assessed by RP-HPLC (see General Procedures)). Sodium sulphate was shown to have a marked impact on physical stability, resulting in considerable precipitation, which prevented further sample analysis by SEC and RP-HPLC.

Table 1: Stability of insulin glargine (100 U/ml) assessed by visual assessment following storage at 30 °C for 4 and 12 weeks. All formulations contained 25 mM m-cresol, 185 mM glycerol and 30 μg/ml ionic zinc and were adjusted to pH 4.0. Extent of visible precipitation is graded on a scale 1-3; 1 = clear solution free of visible particles; 2 = slight particle formation, 3 = more significant precipitation.

| Additive | Visual assessment (0 weeks) | Visual assessment (4 weeks) | Visual assessment (12 weeks) |
|---|---|---|---|
| None | 1 | 1 | 2 |
| Sodium chloride (10 mM) | 1 | 2 | 2 |
| Sodium chloride (50 mM) | 1 | 3 | 3 |
| Sodium sulphate (10 mM) | 1 | 3 | 3 |
| Sodium sulphate (50 mM) | 2 | 3 | 3 |

Table 2: Stability of insulin glargine (100 U/ml) assessed by SEC following storage at 30 °C for 4 and 12 weeks. All formulations contained 25 mM m-cresol, 185 mM glycerol and 30 μg/ml ionic zinc and were adjusted to pH 4.0.

| Additive | SEC main peak (0 weeks) | SEC main peak (4 weeks) | SEC main peak (12 weeks) |
|---|---|---|---|
| None | 99.85% | 99.31% | 98.88% |
| Sodium chloride (10 mM) | 99.90% | 99.24% | 98.56% |
| Sodium chloride (50 mM) | 99.90% | 99.09% | 98.06% |
| Sodium sulphate (10 mM) | - | - | - |
| Sodium sulphate (50 mM) | - | - | - |

Table 3: Stability of insulin glargine (100 U/ml) assessed by RP-HPLC following storage at 30 °C for 4 and 12 weeks. All formulations contained 25 mM m-cresol, 185 mM glycerol and 30 μg/ml ionic zinc and were adjusted to pH 4.0.

| Additive | RP-HPLC main peak (0 weeks) | RP-HPLC main peak (4 weeks) | RP-HPLC main peak (12 weeks) |
|---|---|---|---|
| None | 99.51% | 98.24% | 95.76% |
| Sodium chloride (10 mM) | 99.51% | 98.15% | 95.05% |

(continued)

| Additive | RP-HPLC main peak (0 weeks) | RP-HPLC main peak (4 weeks) | RP-HPLC main peak (12 weeks) |
|---|---|---|---|
| Sodium chloride (50 mM) | 99.50% | 97.11% | 89.53% |
| Sodium sulphate (10 mM) | - | - | - |
| Sodium sulphate (50 mM) | - | - | - |

Example 2 - Effect of an additive on the stability of insulin glargine (100 U/ml) - aspartic acid

The effect of aspartic acid as an additive on the stability of insulin glargine (100 U/ml) was studied by comparing the stability of insulin glargine (100 U/ml) in the currently marketed formulation of Lantus® both in the absence and in the presence of aspartic acid. Aspartic acid was used in the form of a mono-sodium salt. The currently marketed formulation of Lantus® contains 25 mM m-cresol, 185 mM glycerol and 30 $\mu$g/ml ionic zinc and is adjusted to pH 4.0.

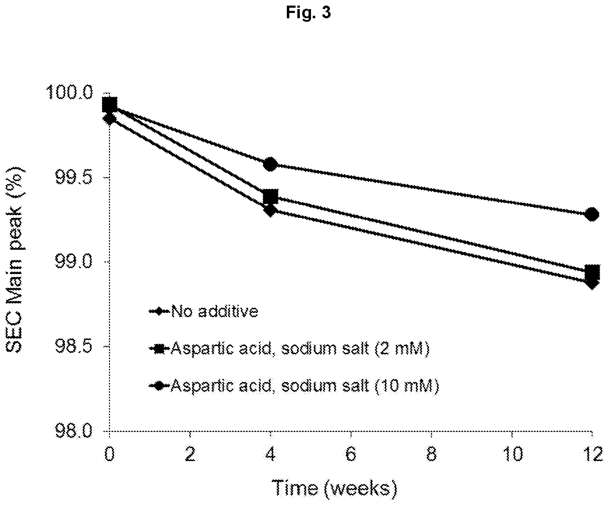

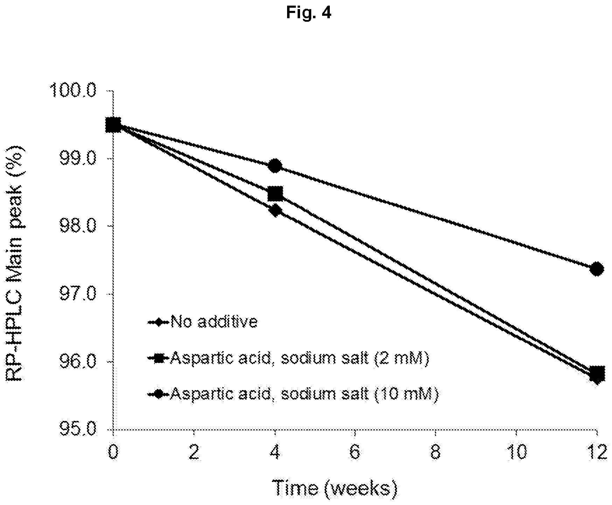

It can be seen from Tables 4-6 below (and Figures 3-4) that the addition of aspartic acid resulted in improvement of both physical stability (assessed by visual assessment and SEC (see General Procedures)) and chemical stability (assessed by RP-HPLC (see General Procedures)) of insulin glargine in the formulation.

Table 4: Stability of insulin glargine (100 U/ml) assessed by visual assessment following storage at 30 °C for 4 and 12 weeks. All formulations contained 25 mM m-cresol and 185 mM glycerol and were adjusted to pH 4.0. Extent of visible precipitation is graded on a scale 1-3; 1 = clear solution free of visible particles; 2 = slight particle formation, 3 = more significant precipitation.

| Additive | Visual assessment (0 weeks) | Visual assessment (4 weeks) | Visual assessment (12 weeks) |
|---|---|---|---|
| None | 1 | 1 | 2 |
| Aspartic acid, mono-sodium salt (2 mM) | 1 | 1 | 1 |
| Aspartic acid, mono-sodium salt (10 mM) | 1 | 1 | 1 |

Table 5: Stability of insulin glargine (100 U/ml) assessed by SEC following storage at 30 °C for 4 and 12 weeks. All formulations contained 25 mM m-cresol, 185 mM glycerol and 30 $\mu$g/ml ionic zinc and were adjusted to pH 4.0.

| Additive | SEC main peak (0 weeks) | SEC main peak (4 weeks) | SEC main peak (12 weeks) |
|---|---|---|---|
| None | 99.85% | 99.31% | 98.88% |
| Aspartic acid, mono-sodium salt (2 mM) | 99.93% | 99.39% | 98.94% |
| Aspartic acid, mono-sodium salt (10 mM) | 99.92% | 99.58% | 99.28% |

Table 6: Stability of insulin glargine (100 U/ml) assessed by RP-HPLC following storage at 30 °C for 4 and 12 weeks. All formulations contained 25 mM m-cresol, 185 mM glycerol and 30 $\mu$g/ml ionic zinc and were adjusted to pH 4.0.

| Additive | RP-HPLC main peak (0 weeks) | RP-HPLC main peak (4 weeks) | RP-HPLC main peak (12 weeks) |
|---|---|---|---|
| None | 99.51% | 98.24% | 95.76% |

(continued)

| Additive | RP-HPLC main peak (0 weeks) | RP-HPLC main peak (4 weeks) | RP-HPLC main peak (12 weeks) |
|---|---|---|---|
| Aspartic acid, mono-sodium salt (2 mM) | 99.50% | 98.49% | 95.82% |
| Aspartic acid, mono-sodium salt (10 mM) | 99.51% | 98.89% | 97.37% |

Example 3 - Effect of an additive on stability of insulin glargine (100 U/ml) - aspartic acid, glutamic acid and glycine

An additional experiment was performed to study the effect of aspartic acid, glutamic acid and glycine as additives on the stability of insulin glargine (100 U/ml). The effect was studied by comparing the stability of insulin glargine (100 U/ml) in the currently marketed formulation of Lantus® both in the absence and in the presence of specific concentrations of each additive. Aspartic acid and glutamic acid were used in the form of mono-sodium salts. The currently marketed formulation of Lantus® contains 25 mM m-cresol, 185 mM glycerol and 30 $\mu$g/ml ionic zinc and is adjusted to pH 4.0.

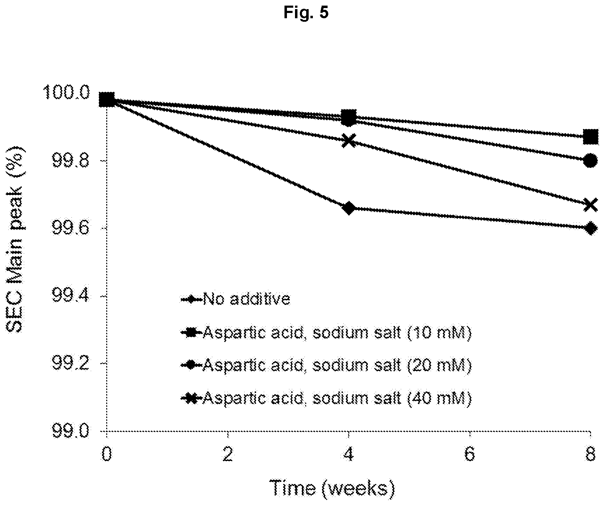

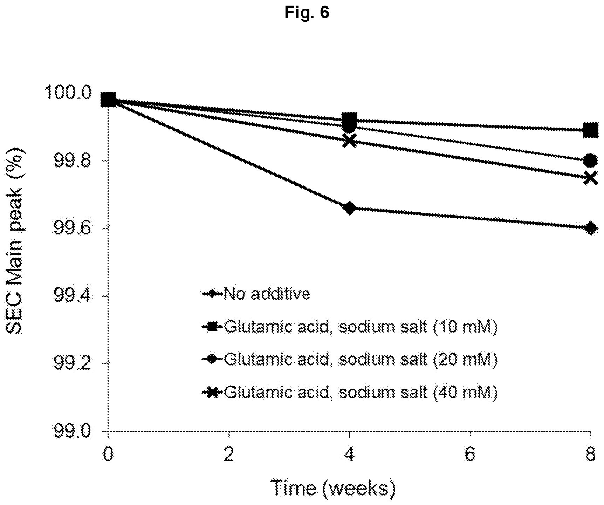

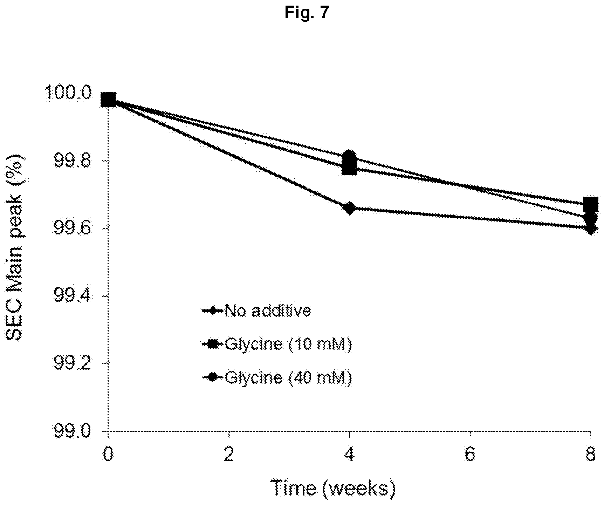

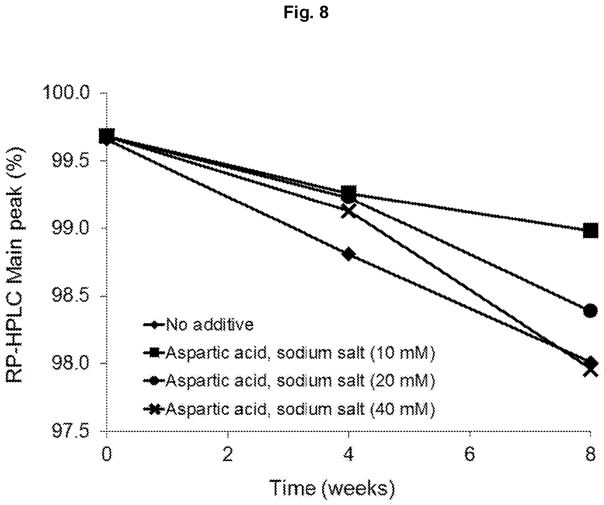

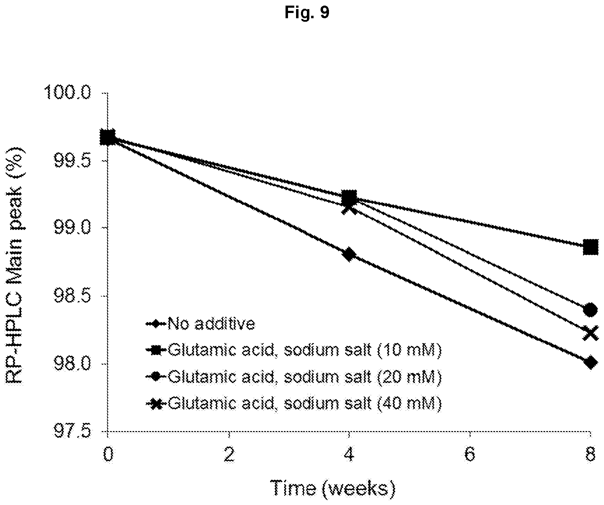

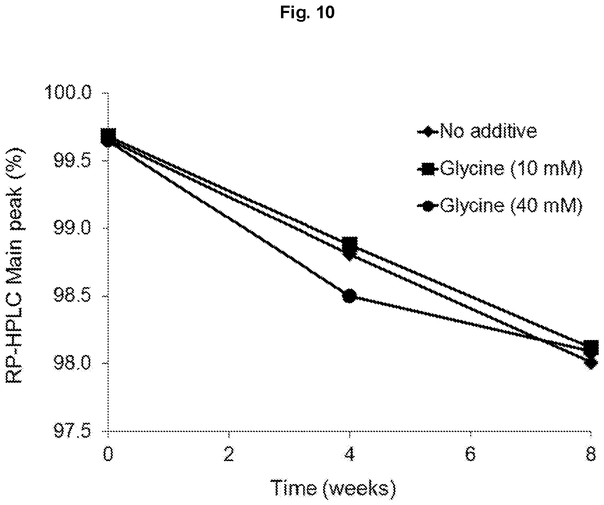

It can be seen from Tables 7-9 below (and Figures 6 and 9) that the addition of glutamic acid resulted in improvement of both physical stability (assessed by visual assessment and SEC (see General Procedures)) and chemical stability (assessed by RP-HPLC (see General Procedures)) of insulin glargine. Confirming the findings of Example 2, an improvement in physical and chemical stability of insulin glargine was also observed on addition of aspartic acid (Figures 5 and 8). In both cases, the magnitude of the stability improvement was greatest if 10 mM of the additive was used compared with higher additive concentrations. In contrast, the addition of glycine as additive did not result in an improvement in physical or chemical stability of insulin glargine comparable to that achieved by aspartic acid and glutamic acid (Figures 7 and 10).

Table 7: Stability of insulin glargine (100 U/ml) assessed by visual assessment following storage at 30 °C for 4 and 8 weeks. All formulations contained 25 mM m-cresol, 185 mM glycerol and 30 $\mu$g/ml ionic zinc and were adjusted to pH 4.0. Extent of visible precipitation is graded on a scale 1-3; 1 = clear solution free of visible particles; 2 = slight particle formation, 3 = more significant precipitation.

| Additive | Visual assessment (0 weeks) | Visual assessment (4 weeks) | Visual assessment (8 weeks) |
|---|---|---|---|
| None | 1 | 1 | 2 |
| Aspartic acid, mono-sodium salt (10 mM) | 1 | 1 | 1 |
| Aspartic acid, mono-sodium salt (20 mM) | 1 | 1 | 1 |
| Aspartic acid, mono-sodium salt (40 mM) | 1 | 1 | 1 |
| Glutamic acid, mono-sodium salt (10 mM) | 1 | 1 | 1 |
| Glutamic acid, mono-sodium salt (20 mM) | 1 | 1 | 1 |
| Glutamic acid, mono-sodium salt (40 mM) | 1 | 1 | 1 |
| Glycine (10 mM) | 1 | 1 | 2 |
| Glycine (40 mM) | 1 | 1 | 2 |

Table 8: Stability of insulin glargine (100 U/ml) assessed by SEC following storage at 30 °C for 4 and 8 weeks. All formulations contained 25 mM m-cresol, 185 mM glycerol and 30 μg/ml ionic zinc and were adjusted to pH 4.0.

| Additive | SEC main peak (0 weeks) | SEC main peak (4 weeks) | SEC main peak (8 weeks) |
|---|---|---|---|
| None | 99.98% | 99.66% | 99.60% |
| Aspartic acid, mono-sodium salt (10 mM) | 99.98% | 99.93% | 99.87% |
| Aspartic acid, mono-sodium salt (20 mM) | 99.98% | 99.92% | 99.80% |
| Aspartic acid, mono-sodium salt (40 mM) | 99.98% | 99.86% | 99.67% |
| Glutamic acid, mono-sodium salt (10 mM) | 99.98% | 99.92% | 99.89% |
| Glutamic acid, mono-sodium salt (20 mM) | 99.98% | 99.90% | 99.80% |
| Glutamic acid, mono-sodium salt (40 mM) | 99.98% | 99.86% | 99.75% |
| Glycine (10 mM) | 99.98% | 99.78% | 99.67% |
| Glycine (40 mM) | 99.98% | 99.81% | 99.63% |

Table 9: Stability of insulin glargine (100 U/ml) assessed by RP-HPLC following storage at 30 °C for 4 and 8 weeks. All formulations contained 25 mM m-cresol, 185 mM glycerol and 30 μg/ml ionic zinc and were adjusted to pH 4.0.

| Additive | RP-HPLC main peak (0 weeks) | RP-HPLC main peak (4 weeks) | RP-HPLC main peak (8 weeks) |
|---|---|---|---|
| None | 99.66% | 98.81% | 98.01% |
| Aspartic acid, mono-sodium salt (10 mM) | 99.68% | 99.26% | 98.98% |
| Aspartic acid, mono-sodium salt (20 mM) | 99.68% | 99.23% | 98.39% |
| Aspartic acid, mono-sodium salt (40 mM) | 99.68% | 99.13% | 97.96% |
| Glutamic acid, mono-sodium salt (10 mM) | 99.67% | 99.23% | 98.86% |
| Glutamic acid, mono-sodium salt (20 mM) | 99.67% | 99.23% | 98.40% |
| Glutamic acid, mono-sodium salt (40 mM) | 99.68% | 99.16% | 98.23% |
| Glycine (10 mM) | 99.68% | 98.88% | 98.12% |
| Glycine (40 mM) | 99.65% | 98.50% | 98.09% |

Example 4 - Effect of aspartic acid on stability of insulin glargine (500 U/ml)

The effect of aspartic acid on the stability of insulin glargine (500 U/ml) was studied by comparing the stability of insulin glargine (500 U/ml) formulated in the currently marketed formulation of Lantus® (Control formulation) both in the absence and in the presence of the aspartic acid. The zinc level in all compositions tested was adjusted to account for the increased concentration of insulin glargine so that the weight ratio between insulin glargine and zinc was the same as that in the marketed Lantus® formulation. The Control formulation thus contained 500 U/ml insulin glargine, 25 mM m-cresol, 185 mM glycerol and 150 μg/ml ionic zinc and was adjusted to pH 4.0.

Tables 10-12 below show that the addition of aspartic acid resulted in improvement of both physical stability (assessed by visual assessment and SEC (see General Procedures)) and chemical stability (assessed by RP-HPLC (see General Procedures)) of insulin glargine. The effect appeared to be more pronounced using 10 mM concentration than 50 mM concentration, particularly with respect to physical stability. Replacing glycerol with trehalose as a tonicity modifier also led to further slight improvement of stability.

Table 10: Stability of insulin glargine (500 U/ml) assessed by visual assessment following storage at 30 °C for 4 and 8 weeks. All formulations contained 25 mM m-cresol and 150 μg/ml ionic zinc and were adjusted to pH 4.0. Extent of visible precipitation is graded on a scale 1-3; 1 = clear solution free of visible particles; 2 = slight particle formation, 3 = more significant precipitation.

| Additive | Visual assessment (0 weeks) | Visual assessment (4 weeks) | Visual assessment (8 weeks) |
|---|---|---|---|
| None | 1 | 2 | 3 |
| Aspartic acid, mono-sodium salt (10 mM) + glycerol (185 mM) | 1 | 1 | 2 |
| Aspartic acid, mono-sodium salt (10 mM) + trehalose (185 mM) | 1 | 1 | 1 |
| Aspartic acid, mono-sodium salt (50 mM) + glycerol (185 mM) | 1 | 2 | 3 |

Table 11: Stability of insulin glargine (500 U/ml) assessed by SEC following storage at 30 °C for 4 and 8 weeks. All formulations contained 25 mM m-cresol and 150 μg/ml ionic zinc and were adjusted to pH 4.0.

| Additive | SEC main peak (0 weeks) | SEC main peak (4 weeks) | SEC main peak (8 weeks) |
|---|---|---|---|
| None | 99.99% | 99.87% | 99.80% |
| Aspartic acid, mono-sodium salt (10 mM) + glycerol (185 mM) | 99.99% | 99.92% | 99.87% |
| Aspartic acid, mono-sodium salt (10 mM) + trehalose (185 mM) | 99.99% | 99.93% | 99.88% |
| Aspartic acid, mono-sodium salt (50 mM) + glycerol (185 mM) | 99.99% | 99.88% | 99.79% |

Table 12: Stability of insulin glargine (500 U/ml) assessed by RP-HPLC following storage at 30 °C for 4 and 8 weeks. All formulations contained 25 mM m-cresol and 150 μg/ml ionic zinc and were adjusted to pH 4.0.

| Additive | RP-HPLC main peak (0 weeks) | RP-HPLC main peak (4 weeks) | RP-HPLC main peak (8 weeks) |
|---|---|---|---|
| None | 99.76% | 99.34% | 98.84% |
| Aspartic acid, mono-sodium salt (10 mM) + glycerol (185 mM) | 99.78% | 99.54% | 99.15% |
| Aspartic acid, mono-sodium salt (10 mM) + trehalose (185 mM) | 99.76% | 99.58% | 99.32% |
| Aspartic acid, mono-sodium salt (50 mM) + glycerol (185 mM) | 99.78% | 99.40% | 99.16% |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The invention embraces all combinations of preferred and more preferred groups and suitable and more suitable groups and embodiments of groups recited above.

SEQUENCE LISTING

SEQ ID NO. 1:

Chain A: NH2-Gly-Ile-Val-Glu-Gln-Cys-Cys-Thr-Ser-Ile-Cys-Ser-Leu-Tyr-Gln-Leu-Glu-Asn-Tyr-Cys-Gly-COOH

SEQ ID NO. 2:

Chain B: NH2-Phe-Val-Asn-Gln-His-Leu-Cys-Gly-Ser-His-Leu-Val-Glu-Ala-Leu-Tyr-Leu-Val-Cys-Gly-Glu-Arg-Gly-Phe-Phe-Tyr-Thr-Pro-Lys-Thr-Arg-Arg-COOH

**Claims**

1. An aqueous solution composition comprising insulin glargine as an active ingredient and an amino acid selected from aspartic acid and glutamic acid or wherein the amino acid is a mixture thereof as a stabilising agent, wherein the amino acid is present at a concentration of 1-50 mM.

2. An aqueous solution composition according to claim 1, wherein the concentration of insulin glargine is between 10 U/ml and 1000 U/ml, for example between 50 U/ml and 500 U/ml, or between 100 U/ml and 200 U/ml; or wherein the concentration of insulin glargine is between 200 U/ml and 500 U/ml.

3. An aqueous solution composition according to claim 1 or claim 2, wherein the amino acid is aspartic acid; or

   wherein the amino acid is glutamic acid; or
   wherein the amino acid is a mixture of aspartic acid and glutamic acid.

4. An aqueous solution composition according to any one of claims 1 to 3, wherein the concentration of amino acid is 2-45 mM, 5-40 mM or 2-25 mM, such as 5-15 mM or 7-12 mM, for example 10 mM; and/or
   wherein the pH is between 3 and 5, such as pH 4.

5. An aqueous solution composition according to any one of claims 1 to 4, further comprising a tonicity modifier which is suitably an uncharged tonicity modifier and is selected from glycerol, 1,2-propanediol, mannitol, sorbitol, trehalose, PEG300 and PEG400.

6. An aqueous solution composition according to any one of claims 1 to 5, further comprising a surfactant.

7. An aqueous solution composition according to claim 6, wherein the surfactant is a non-ionic surfactant.

8. An aqueous solution composition according to claim 6, wherein the surfactant is a cationic surfactant, wherein the cationic surfactant is suitably selected from a benzalkonium salt and a benzethonium salt; or

   wherein the cationic surfactant is suitably selected from benzethonium salts such as benzethonium chloride; or
   wherein the cationic surfactant is suitably selected from benzalkonium salts such as benzalkonium chloride; or
   wherein the cationic surfactant is suitably a mixture of benzethonium salts and benzalkonium salts such as a mixture of benzethonium chloride and benzalkonium chloride.

9. An aqueous solution composition according to any one of claims 1 to 8, which additionally comprises a preservative such as a phenolic or benzylic preservative, wherein the phenolic or benzylic preservative is suitably selected from the group consisting of phenol, m-cresol, chlorocresol, benzyl alcohol, propyl paraben and methyl paraben.

10. An aqueous solution composition according to any one of claims 1 to 9, which is a therapeutic composition.

11. An aqueous solution composition according to any one of claims 1 to 10, for use in the treatment of a subject suffering from diabetes mellitus.

12. Use of an amino acid selected from aspartic acid and glutamic acid, or wherein the amino acid is a mixture thereof at a concentration of 1-50 mM, for stabilizing an aqueous solution composition of insulin glargine as an active ingredient.

13. A method of improving the stability of an aqueous solution composition comprising insulin glargine as an active ingredient which comprises adding an amino acid selected from aspartic acid and glutamic acid or wherein the amino acid is a mixture thereof as a stabilising agent, at a concentration of 1-50 mM to the composition.

14. A container containing one dose or a plurality of doses of an aqueous solution composition according to any one of claims 1 to 10, wherein the container is suitably a vial.

15. An injection device for single or multiple-use comprising a container according to claim 14 together with an injection needle, wherein the injection device is suitably in the form of a pen.

**Patentansprüche**

1. Wässrige Lösungszusammensetzung, umfassend Insulin glargin als ein Wirkstoff und eine Aminosäure ausgewählt aus Asparaginsäure und Glutaminsäure, oder wobei es sich bei der Aminosäure um ein Gemisch davon handelt, als ein Stabilisierungsmittel, wobei die Aminosäure in einer Konzentration von 1 - 50 mM vorliegt.

2. Wässrige Lösungszusammensetzung nach Anspruch 1, wobei die Konzentration von Insulin glargin zwischen 10 U/ml und 1000 U/ml liegt, zum Beispiel zwischen 50 U/ml und 500 U/ml oder zwischen 100 U/ml und 200 U/ml; oder wobei die Konzentration von Insulin glargin zwischen 200 U/ml und 500 U/ml liegt.

3. Wässrige Lösungszusammensetzung nach Anspruch 1 oder Anspruch 2, wobei es sich bei der Aminosäure um Asparaginsäure handelt; oder wobei es sich bei der Aminosäure um Glutaminsäure handelt; oder wobei es sich bei der Aminosäure um ein Gemisch von Asparaginsäure und Glutaminsäure handelt.

4. Wässrige Lösungszusammensetzung nach einem der Ansprüche 1 bis 3, wobei die Konzentration der Aminosäure 2 - 45 mM, 5 - 40 mM oder 2 - 25 mM beträgt, wie 5 - 15 mM oder 7 - 12 mM, zum Beispiel 10 mM; und/oder wobei der pH-Wert zwischen 3 und 5, wie pH-Wert 4, liegt.

5. Wässrige Lösungszusammensetzung nach einem der Ansprüche 1 bis 4, ferner umfassend einen Tonizitätsmodifizierer, der geeigneterweise ein ungeladener Tonizitätsmodifizierer ist und der aus Glycerin, 1,2-Propandiol, Mannit, Sorbit, Trehalose, PEG300 und PEG400 ausgewählt wird.

6. Wässrige Lösungszusammensetzung nach einem der Ansprüche 1 bis 5, ferner umfassend ein Tensid.

7. Wässrige Lösungszusammensetzung nach Anspruch 6, wobei es sich bei dem Tensid um ein nichtionisches Tensid handelt.

8. Wässrige Lösungszusammensetzung nach Anspruch 6, wobei es sich bei dem Tensid um ein kationisches Tensid handelt, wobei das kationische Tensid geeigneterweise aus einem Benzalkoniumsalz und einem Benzethoniumsalz ausgewählt wird, oder

   wobei das kationische Tensid geeigneterweise aus Benzethoniumsalzen wie Benzethoniumchlorid ausgewählt wird; oder
   wobei das kationische Tensid geeigneterweise aus Benzalkoniumsalzen wie Benzalkoniumchlorid ausgewählt wird; oder
   wobei es sich bei dem kationischen Tensid geeigneterweise um ein Gemisch aus Benzethoniumsalzen und Benzalkoniumsalzen wie ein Gemisch aus Benzethoniumchlorid und Benzalkoniumchlorid handelt.

9. Wässrige Lösungszusammensetzung nach einem der Ansprüche 1 bis 8, die zusätzlich ein Konservierungsmittel wie ein phenolisches oder benzylisches Konservierungsmittel umfasst, wobei das phenolische oder benzylische Konservierungsmittel geeigneterweise aus der Gruppe bestehend aus Phenol, m-Kresol, Chlorkresol, Benzylalkohol, Propylparaben und Methylparaben ausgewählt wird.

10. Wässrige Lösungszusammensetzung nach einem der Ansprüche 1 bis 9, die eine therapeutische Zusammensetzung ist.

11. Wässrige Lösungszusammensetzung nach einem der Ansprüche 1 bis 10 zur Verwendung bei der Behandlung eines Individuums, das an Diabetes mellitus leidet.

**12.** Verwendung einer Aminosäure, ausgewählt aus Asparaginsäure und Glutaminsäure, oder wobei es sich bei der Aminosäure um ein Gemisch davon handelt, in einer Konzentration von 1 - 50 mM zum Stabilisieren einer wässrigen Lösungszusammensetzung von Insulin glargin als ein Wirkstoff.

**13.** Verfahren zur Verbesserung der Stabilität einer wässrigen Lösungszusammensetzung, die Insulin glargin als ein Wirkstoff umfasst, das das Hinzufügen einer Aminosäure, die aus Asparaginsäure und Glutaminsäure ausgewählt wird, oder wobei es sich bei der Aminosäure um ein Gemisch davon handelt, als ein Stabilisierungsmittel in einer Konzentration von 1 - 50 mM zu der Zusammensetzung umfasst.

**14.** Behälter, enthaltend eine Dosis oder eine Vielzahl von Dosen einer wässrigen Lösungszusammensetzung nach einem der Ansprüche 1 bis 10, wobei es sich bei dem Behälter geeigneterweise um ein Fläschchen handelt.

**15.** Injektionsvorrichtung zur einmaligen oder mehrfachen Verwendung, umfassend einen Behälter nach Anspruch 14 zusammen mit einer Injektionsnadel, wobei es sich bei der Injektionsvorrichtung geeigneterweise um einen Pen handelt.

**Revendications**

**1.** Composition de solution aqueuse comprenant de l'insuline glargine en tant qu'ingrédient actif et un acide aminé choisi parmi l'acide aspartique et l'acide glutamique, ou l'acide aminé étant un mélange de ceux-ci, en tant qu'agent stabilisant, l'acide aminé étant présent à une concentration de 1 à 50 mM.

**2.** Composition de solution aqueuse selon la revendication 1, la concentration d'insuline glargine étant comprise entre 10 U/ml et 1 000 U/ml, par exemple entre 50 U/ml et 500 U/ml, ou entre 100 U/ml et 200 U/ml ; ou la concentration d'insuline glargine étant comprise entre 200 U/ml et 500 U/ml.

**3.** Composition de solution aqueuse selon la revendication 1 ou la revendication 2, l'acide aminé étant l'acide aspartique ; ou

l'acide aminé étant l'acide glutamique ; ou
l'acide aminé étant un mélange d'acide aspartique et d'acide glutamique.

**4.** Composition de solution aqueuse selon l'une quelconque des revendications 1 à 3, la concentration d'acide aminé étant de 2 à 45 mM, de 5 à 40 mM ou de 2 à 25 mM, telle que de 5 à 15 mM ou de 7 à 12 mM, par exemple de 10 mM ; et/ou le pH étant compris entre 3 et 5, tel que pH 4.

**5.** Composition de solution aqueuse selon l'une quelconque des revendications 1 à 4, comprenant en outre un modificateur de tonicité qui est de manière appropriée un modificateur de tonicité non chargé et est choisi parmi le glycérol, le 1,2-propanediol, le mannitol, le sorbitol, le tréhalose, un PEG300 et un PEG400.

**6.** Composition de solution aqueuse selon l'une quelconque des revendications 1 à 5, comprenant en outre un tensioactif.

**7.** Composition de solution aqueuse selon la revendication 6, le tensioactif étant un tensioactif non ionique.

**8.** Composition de solution aqueuse selon la revendication 6, le tensioactif étant un tensioactif cationique, le tensioactif cationique étant choisi de manière appropriée parmi un sel de benzalkonium et un sel de benzéthonium ; ou

le tensioactif cationique étant choisi de manière appropriée parmi des sels de benzéthonium tels que le chlorure de benzéthonium ; ou
le tensioactif cationique étant choisi de manière appropriée parmi des sels de benzalkonium tels que le chlorure de benzalkonium ; ou
le tensioactif cationique étant de manière appropriée un mélange de sels de benzéthonium et de sels de benzalkonium tel qu'un mélange de chlorure de benzéthonium et de chlorure de benzalkonium.

**9.** Composition de solution aqueuse selon l'une quelconque des revendications 1 à 8, qui comprend de plus un conservateur tel qu'un conservateur phénolique ou benzylique, le conservateur phénolique ou benzylique étant choisi de manière appropriée dans le groupe constitué par le phénol, le m-crésol, le chlorocrésol, l'alcool benzylique, le propylparabène et le méthylparabène.

10. Composition de solution aqueuse selon l'une quelconque des revendications 1 à 9, qui est une composition thérapeutique.

11. Composition de solution aqueuse selon l'une quelconque des revendications 1 à 10, pour une utilisation dans le traitement d'un sujet souffrant du diabète sucré.

12. Utilisation d'un acide aminé choisi parmi l'acide aspartique et l'acide glutamique, ou l'acide aminé étant un mélange de ceux-ci, à une concentration de 1 à 50 mM, pour la stabilisation d'une composition de solution aqueuse d'insuline glargine en tant qu'ingrédient actif.

13. Procédé d'amélioration de la stabilité d'une composition de solution aqueuse comprenant de l'insuline glargine en tant qu'ingrédient actif qui comprend l'ajout d'un acide aminé choisi parmi l'acide aspartique et l'acide glutamique, ou l'acide aminé étant un mélange de ceux-ci, en tant qu'agent stabilisant, à une concentration de 1 à 50 mM à la composition.

14. Récipient contenant une dose ou une pluralité de doses d'une composition de solution aqueuse selon l'une quelconque des revendications 1 à 10, le récipient étant de manière appropriée un flacon.

15. Dispositif d'injection pour une utilisation unique ou multiple comprenant un récipient selon la revendication 14 conjointement avec une aiguille d'injection, l'aiguille d'injection étant de manière appropriée sous la forme d'un crayon.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

**Fig. 11**

Fig. 12

## REFERENCES CITED IN THE DESCRIPTION

**Patent documents cited in the description**

- WO 2010149772 A
- WO 2014096985 A
- US 2015273022 A
- US 2008039368 A
- US 2011230402 A
- WO 2017145104 A